# EUROPÄISCHE PATENTSCHRIFT

(11) **EP 0 944 831 B1**
(45) Veröffentlichungstag und Bekanntmachung des Hinweises auf die Patenterteilung: **28.08.2002**
(21) Anmeldenummer: 97952868.4
(22) Anmeldetag: 03.12.1997
(51) Int. Cl.: G01N 33/53, G01N 33/36

(54) **IMMUNOCHEMISCHE BESTIMMUNG VON INHALTSSTOFFEN AUF TEXTILFASERN ODER POLYMEREN**
IMMUNOCHEMICAL DETERMINATION OF SUBSTANCES CONTAINED IN TEXTILE FIBRES OR POLYMERS
DETERMINATION IMMUNOCHIMIQUE DES SUBSTANCES CONTENUES DANS DES FIBRES TEXTILES OU AUTRES POLYMERES

(30) Priorität: 11.12.1996 DE 19651599
(43) Veröffentlichungstag der Anmeldung: 29.09.1999
(73) Patentinhaber: Diagnostic Systems Biotechnologie GmbH, 80801 München (DE)
(72) Erfinder: LANGHALS, Heinz, 85521 Ottobrunn (DE); BROSIUS, Rupert, 80801 Munchen (DE); HOCK, Bertold, 85354 Freising (DE)
(74) Vertreter: Turi, Michael, Dipl.-Phys.
(86) Internationale Anmeldenummer: EP9706763
(87) Internationale Veröffentlichungsnummer: WO98026285

(56) Entgegenhaltungen:
- US-A- 5 324 642
- DEWAIR M ET AL: "Use of immunoblot technique for detection of human IgE and IgG antibodies to individual silk proteins." THE JOURNAL OF ALLERGY AND CLINICAL IMMUNOLOGY, Bd. 76, Nr. 4, Oktober 1985, ST. LOUIS, MO, Seiten 537-542, XP002061711
- HAUSEN B.M. ET AL: "Strumpffarben-Allergie" DEUTSCHE MEDIZINISCHE WOCHENSCHRIFT, Bd. 109, Nr. 39, 29.September 1984, STUTTGART, Seiten 1469-1475, XP002061712

## Beschreibung

Die vorliegende Erfindung betrifft ein Verfahren zum Nachweis von Inhaltsstoffen auf Textilfasern oder anderen natürlichen oder synthetischen Polymeren.

Bspw. Textilien oder auch Nahrungs- und Genußmitteln weisen zahlreiche Inhaltsstoffe auf, die beim Konsumenten je nach immunologischer Disposition als Allergene wirken können. Diese Allergene lösen allergische Reaktionen aus, die sich in einer umfangreichen Symptomatik, zumeist durch eine starke Histamin-Aussschüttung bedingt, manifestieren. Erytheme, Schleimhautreizungen, asthmatische Reaktionen oder auch Blutdruckabfall mit schockähnlichen Zuständen können für den Allergiker die Folge sein. Es ist daher für den Allergiker von größter Bedeutung, in Form eines schnell und einfach vorzunehmenden Verfahrens, solche Substanzen in Textilien, Nahrungs- und Genußmitteln etc. zu identifizieren, die gemäß dem jeweiligen individuellen allergischen Profil ein Risikopotential darstellen.

Die vorliegende Erfindung offenbart ein Verfahren zum Nachweis bzw. zur quantitativen Bestimmung von Inhaltsstoffen von Textilfasern und/oder von anderen natürlichen oder synthetischen Polymeren mit Hilfe von Antikörpern. Verfahrensgemäß kann hier der Konsument selbst vor dem Erwerb das allergene Potential einer Textilie oder auch eines Nahrungs- oder Genußmittels überprufen.

Die US-A-5 324 642 beschreibt ein Verfahren zur Analyse eines Analyten in einer Keratin-Struktur (Haare, Nägel), in welchem man ein Enzym und eine Verbindung mit niedrigem Redox-Potential auf eine Probe der Keratin-Struktur einwirken läßt, um dieselbe abzubauen und den Analyten in dieser Verdauungslösung zu lösen. Der Nachweis des Analyten erfolgt bevorzugt durch Immunassaytechniken auf Protein-Basis (d.h. mit Antikörpern).

In Dewair et al., J. Allergy Clin. Immunol. 76 (4), 537-542 (1985) "Use of immunoblot technique for detection of human IgE and IgG antibodies to individual silk proteins", werden Seideproteine aus Seide extrahiert, mittels Polyacrylamid-Gelelektrophorese (also nicht mit Antikörpern) aufgetrennt und mit menschlichem Serum inkubiert, das IgE- und IgG-Antikörper gegen Seideproteine enthalten kann. Der Nachweis der an Seideproteine gebundenen Antikörper wird mittels Immunoblot-Technik geführt.

In B. M. Hausen et al., Deutsche Medizinische Wochenschrift, 109 (39), 1469-1475 (1984) "Strumpffarben-Allergie" wird berichtet, daß Testpersonen mit Strumpfallergie auf isolierte, mittels Chromatographie aufgetrennte Strumpffarben (insbesondere Azofarbstoffe) im Epikutantest allergisch reagierten. Ein Nachweis der Allergene mittels Antikörper ist nicht beschrieben.

Der besondere Vorteil der zum Nachweis verfahrensgemaß eingesetzten Reagenzien, nämlich der Antikörper, liegt darin begründet, daß sie nicht nur an gelöste Moleküle binden, sondern auch an immobilisierte Strukturen. Dies eröffnet die attraktive Möglichkeit, Inhaltsstoffe, die als Allergene wirken können, auch direkt auf der Faser oder dem Polymer zu bestimmen. Hierdurch entfallen die aufwendigen und mit analytischen Unsicherheiten behafteten Desorptionen oder Abspaltungen der Inhaltsstoffe. Daher wird in der vorliegenden Erfindung die Nachweisreaktion direkt auf den Fasern einer Textilie oder auf natürlichen oder synthetischen Polymeren durchgerührt. Hier erfolgt also der direkte Nachweis des potentiellen allergenen Inhaltsstoffs mit Hilfe einer direkten Bestimmung des Antikörpers auf Faser oder Polymer.

In einer keinesfalls abschließenden Aufzählung von Faser- oder Polymer-Materialien, deren Inhaltsstoffe erfindungsgemäß nachgewiesen werden können, seien beispielsweise Baumwolle, Wolle oder anderes Tierhaar, so z.B. Roßhaar, Seide, Jute, Sisal, Hanf oder Flachs und deren Umwandlungsprodukte wie z.B. die Viskosefaser, Nitratseide oder Kupferrayon (Reyon) oder synthetische Materialien aus Polyvinylchlorid, Celluloseacetat, Polycarbonate, Polyamide, Polyurethane, Polyimide, Polybenzimi-dazole, Melaminharze, Silikone, Polyester, Polyether, Polystyrol, Polymethylmethacrylat, Polyethylen, Polypropylen, Polyvinylacetat, Polyacrylnitril, Polybutadien, Polychlorbutadien oder Polyisopren bzw. die Copolymeren der genannten Monomeren oder auch Kombinationen der vorgenannten Polymeren genannt.

Bevorzugt ist weiterhin der Einsatz eines unspezifischen Antikörpers, der dazu dient, die Oberfläche des zu untersuchenden Substrats abzusättigen und damit die unspezifische Bindung der spezifischen Nachweis-Antikörper zu minimieren. Es ist nämlich anzunehmen, daß Fasern oder Polymere vielfältige unspezifische Bindungsmöglichkeiten für die Nachweis-Antikörper besitzen, wodurch die eigentliche Nachweisreaktion gestört wird. Um dies zu unterdrücken, ist es günstig, die Oberfläche des zu untersuchenden Materials mit solchen Antikörpern abzusättigen, die nicht gegen die nachzuweisende Substanz gerichtet sind. Da von diesen Antikörpern verhältnismäßig große Mengen benötigt werden, sollten sie, insbesondere im Falle von polyklonalen Antikörpern, bevorzugt von Kaninchen, Schaf, Pferd, Rind oder Ziege stammen, am stärksten bevorzugt wird jedoch von Schaf. Der Einsatz des unspezifischen Antikörpers erfolgt typischerweise vor der Zugabe des spezifischen Antikörpers.

In einer besonders bevorzugten Ausführungsform stammen diese unspezifischen Antikörper von einem anderen Tier als die spezifischen Antikörper. Spezifische und unspezifische Antikörper unterscheiden sich insofern auch durch ihre sogenannte "konstante Region" F_{c}. Dies erlaubt eine etwaige nachfolgende immunochemische Reaktion mit einem Sekundärantikörper, der bestimmte artspezifische Abschnitte in der konstanten Region der spezifischen Nachweis-Antikörper erkennt. Bei vorgenanntem immunochemischen Nachweis der spezifischen Antikörper darf dieser natürlich nicht von der gleichen Tier-Spezies stammen wie die zur Absättigung benötigten Antikörper.

Die Absättigung mit einem unspezifischen Antikörper ist sowohl im Fall der direkten als auch im Fall der indirekten Bestimmung der Bindung des spezifischen Antikörpers denkbar.

In einer bevorzugten Ausführungsform werden für die Bestimmung der Inhaltsstoffe monoklonale oder polyklonale Antikörper oder aber F_{ab}-Fragmente eingesetzt. Für eine überschlägige Schnellbestimmung wird man bevorzugt die kostengünstigen polyklonalen Antikörper einsetzen, während die monoklonalen Antikörper insbesondere dann geeigneter sind, wenn man genau definierte Kreuzreaktivitäten, z.B im Sinne einer Gruppenselektivität, einstellen möchte. F_{ab}-Fragmente sind vor allem zur Kupplung mit bestimmten Immunolabeln geeignet, außerdem weisen sie eine vergleichsweise geringe Neigung zur unspezifischen Bindung auf.

Beim dem Einsatz von Antikörpern in der Analytik muß zwischen hoch-selektiven, gleichzeitig stark bindenden und wenig selektiven, gleichzeitig schwach bindenden Antikörpern unterschieden werden (mit der Hybridom-Technik können die entsprechenden Antikörper selektiert werden) werden. Während eine hohe Selektivität von Antikörpern geradezu ideal für die Identifizierung einer bestimmten Substanz - hier eines bestimmten Inhaltsstoffs, ggf. eines Allergens - ist, bietet ein wenig selektiver Antikörper den Vorteil einer erheblichen "Kreuzreaktivität", d.h. er bindet an ähnliche Strukturen mit einer ähnlichen Bindungskonstante.

Mit Hilfe der Bindungsstärke der Antikörper kann damit die analytische Empfindlichkeit eingestellt werden. Dies ist für den praktischen Einsatz eines Tests wichtig, da man je nach den gegebenen Erfordernissen eine mehr oder weniger hohe Empfindlichkeit benötigt.

In einer weiteren bevorzugten Ausführungsform wird der Antikörper durch die Immunstimulierung von Säugern, insbesondere Ziegen, Schafen, Schweinen, Ratten, Pferde, Kaninchen, Meerschweinchen, Huhn, Rind und/oder Mäusen, isoliert. Dies geschieht, indem dem Säuger ein bestimmter, ggf. allergener Inhaltsstoff oder Epitope eines bestimmten, ggf. allergenen Inhaltsstoffs mit ausreichenden antigenen Eigenschaften injiziert werden. Der Säuger antwortet auf diese Fremdantigene mit einer Immunreaktion, die zur Bildung von Antikörpern führt. Bei diesem Vorgehen können polyklonale Antikörper gegen das Fremdantigen erhalten werden.

Ein verfahrensgemäß eingesetzter, monoklonaler Antikörper (z.B. Anti-Allergen-Antikörper) wird nach konventionellen, dem Fachmann geläufigen Verfahren hergestellt. Hierzu werden die Milzzellen der immunstimulierten Maus nach Erreichen des optimalen Antikörpertiters mit *in vitro* gezüchteten Myelomzellen in Polyethylenglykol fusioniert und danach in einem HAT-Medium aufgezogen. In diesem Medium können nur sogenannte Hybridomzellen aus Milz und Myelom überleben. Um die geeigneten, Antikörper produzierenden Klone zu isolieren, werden die einzelnen Klone auf ihre Immunoglobulinproduktion überprüft und die produzierenden Klone entweder bevorzugt *in vitro* oder ggf. in Mäusen aufgezüchtet. Für weitere Einzelheiten der Produktion monoklonaler Antikörper wird auf "Monoclonal Antibodies, Hybridomas: A new dimension in biological analyses, Kennet et al., Plenum Press, New York, 1980", "Antibodies, A Laboratory Manual, Harlow & Lane, Cold Spring Harbor Laboratory Press, 1988" und "Peters, J.H., Baumgarten, H., Monoclonal Antibodies 2. Auflage, Springer-Verlag, Berlin-Heidelberg-New York, 1990" Bezug genommen. Die resultierenden monoklonalen Antikörper haben die Eigenschaft, von einer Zelle abzustammen, und weisen somit alle die gleichen spezifischen Antikörpereigenschaften auf, d. h. sie erkennen alle den gleichen Epitop.

Erfindungsgemäße Antikörper können nach den üblichen Verfahren isoliert und gereinigt werden (Antibodies, A Laboratory Manual, Harlow & Lane, Cold Spring Harbor Laboratory Press, 1988). In einer keineswegs abschließenden Aufzählung seien die folgenden Methoden genannt: Peptid- und Proteinsäulenchromatographie, HPLC und "reversed phase"-HPLC, Proteinisolierung auf Protein A- oder Protein G-Säulen oder alle denkbaren Kombinationen dieser Methoden.

In einer bevorzugten Ausführungsform werden die Antikörper für die Nachweisreaktion so markiert, daß ihre Assoziation mit dem Allergen oder der Fremdsubstanz sichtbar gemacht wird. Markermoleküle dienen zur Markierung der spezifischen Nachweis-Antikörper. Auf diese Weise gelingt es, die Präsenz des vom Antikörper erkannten Antigens qualitativ und/oder quantitativ zu bestimmen. Dies ist zum einen durch direkte Markierung des gegen einen ggf. allergenen Inhaltsstoff gerichteten Antikörpers möglich (z.B. durch Fluoreszenzmarkierung, radioaktive Markierung oder die kovalente Kopplung an ein geeignetes Markerenzym), oder auch indirekt durch einen zweiten gegen den spezifischen Anti-Antigen-Antikörper gerichteten Anti-Anti-Antigen-Antikörper, der dann gleichfalls nach den oben beschriebenen oder anderen, dem Fachmann jederzeit geläufigen Verfahren markiert sein kann. Ein Verfahren, bei dem der Nachweis-Antikörper (monoklonal, polyklonal oder als F_{ab}-Fragment) oder ein gegen den Nachweis-Antikörper gerichteter Sekundär-Antikörper fluoreszenzmarkiert sind, sind dabei bevorzugt. In diesem Fall kann bei Bestrahlung mit einer entsprechenden Anregungswellenlänge unmittelbar der Nachweis auf visueller Basis erfolgen.

Das fluoreszierende Molekül kann über Haupt- oder Nebenvalenzen an einen verfahrensgemäß eingesetzten Antikörper gebunden sein.

Bei entsprechender optischer Anregung gibt der Antikörper dann ein Fluoreszenzsignal ab und indiziert damit die Präsenz des jeweiligen Antigens.

Als geeignete Fluoreszenzsonden seien beispielhaft 1-Anilino-8-Naphthalinsulfonat, 1-Dimethylaminonaphthalin-5-sulfonylchlorid und Fluoreszein-Isothiocyanat, Rhodamin bzw. deren Derivate genannt oder Donor-substituierter Oxindigo (Angew. Chem. 1996, 108, 1090-1093), die über Haupt- oder Nebenvalenzen mit den Antikörpern in an und für sich bekannter Weise verknüpft werden können.

Neben der Fluoreszenzmarkierung wird in einer anderen bevorzugten Ausführungsform auch eine Markierung der Anti-Antigen-Antikörper mit radioaktiven Isotopen vorgenommen. Bevorzugt ist der Einsatz des γ-Strahlers ¹²⁵I, mit dem eine Phenolgruppe eines Tyrosins eines erfindungsgemäßen Antikörpers markiert wird. In diesem Fall würde ein Szintigramm zum Nachweis des zu bestimmenden Antigens erstellt. Denkbar ist aber auch, daß weiche β-Strahler, wie ³H, ³⁵S oder ¹⁴C Verwendung finden.

Darüber hinaus kann in einer anderen Ausführungsform das Markermolekül auch ein Enzym darstellen, das ein Substrat auf quantifizierbare Weise umsetzt. Die Enzyme sind kovalent an den Antikörper gebunden. Typischerweise werden als Enzyme die Alkalische Phosphatase, die Peroxidase, insbesondere Meerrettich-Peroxidase, die dann z.B. über die Oxydation mit H₂O₂ und in Gegenwart von Tetramethylbenzidin, nachgewiesen werden kann, indem sich ein blauer Farbstoff bildet, oder Doppelenzymsysteme, bei denen das Produkt der ersten Enzymreaktion (des kovalent an den Antikörper gebundenen Enzyms) durch eine zweite, nachgeschaltete Enzymreaktion weiter umgesetzt und erst das Produkt der Zweitreaktion nachgewiesen wird.

Unter den Inhaltsstoffen von Fasern oder Polymeren, die als Antigene mit Hilfe von Antikörpern nachgewiesen werden und als Allergene oder Fremdsubstanzen in Betracht kommen, werden insbesondere Textilfarbstoffe, Textilhilfsmittel und/oder deren Beistoffe und die Faserstoffe oder Poymere selbst genannt. In einer besonders bevorzugten Ausführungsform der vorliegenden Erfindung werden daher die vorgenannten Inhaltsstoffe mit Hilfe von Antikörpern nachgewiesen.

Die vorgenannten Stoffe sind zwar für die überwiegende Mehrheit der Bevölkerung unbedenklich, für den Kreis der Allergiker stellen sie aber unter Umständen eine erhebliche gesundheitliche Beeinträchtigung dar. Da nicht damit zu rechnen ist, daß wegen eines so kleinen Kreises der Betroffenen ein Verbot dieser Substanzen ausgesprochen wird, ergibt sich daraus auch langfristig ein permanenter Bedarf für ein kostengünstiges Analyseverfahren, das vom Allergiker routinemäßig beim Kauf jedes neuen Kleidungsstück angewandt werden kann.

Unter Textilhilfsmitteln bzw. deren Beistoffen sind solche Substanzen zu verstehen, die bei textilen Prozessen benötigt werden (Gewinnung und Herstellung von Textilfasern, Verarbeitung, Veredlung und Konfektionierung). Folgende, keineswegs abschließende Aufzählung von Beispielen sei genannt: Antistatika, antimikrobielle Substanzen, optische Aufheller, Fraßschutzmittel, Appreturmittel, Imprägniermittel, Stabilisatoren, Steifungsmittel und Mittel für die Textilbeschichtung.

Auch Farbmittel werden bei textilen Prozessen eingesetzt. Sie kommen gemeinsam mit Farbstofflösungs- und Dispergiermitteln zum Einsatz. Als Farbmittel seien Reaktiv-, Direkt-, Küpen-, Schwefel, kationische Kupplungs- und Dispersionsfarbstoffe genannt.

Als Allergene oder auf andere Weise gesundheitlich bedenkliche Substanzen, die erfindungsgemäß nachgewiesen werden, kommen insbesondere jene Inhaltsstoffe in Betracht, die entweder beim Färbeprozeß auf die Faser aufgebracht werden oder aber als Textilhilfsstoffe verwendet werden. Beispiele sind insbesondere Pentachlorphenol (PCP), o-Phenylendiamin, p-Phenylendiamin, Dimethyloldihydroxyethylenharnstoff, Dimethylolethylenharnstoff, Dimethyloltriazon oder Dimethylolharnstoff.

Erfindungsgemäß werden als Inhaltsstoffe ganz besonders bevorzugt Farbstoffe auf Textil-Fasern oder -Polymeren nachgewiesen. Deren Detektion mit Hilfe eines Antikörpers und von bevorzugteingesetzten Fluoreszenzmarkierungen erfordert aber eine Reihe von spezifischen Randbedingungen, die im folgenden behandelt werden:

Es muß zunächst darauf geachtet werden, daß die Anregungsenergie des Fluoreszenzmarkers auch tatsächlich als Fluoreszenzlicht wieder abgestrahlt und nicht etwa auf den Farbstoff übertragen wird und so verloren geht (Fluoreszenzlöschung). Man könnte daran denken, daß man die Anregungswellenlänge des Fluoreszenz-markers länger wählt als die Absorptionswellenlängen der nachzuweisenden Farbstoffe, so daß eine Energieübertragung nicht mehr möglich ist. Ein solcher Weg würde allerdings die Zahl der detektierbaren Farbtöne stark einschränken und würde bei türkisfarbenen Farbstoffen gänzlich versagen, da diese an der langwellig sichtbaren Grenze absorbieren, so daß ein hierfür geeigneter Fluoreszenzmarker im nicht-sichtbaren, sondern im NIR-Bereich fluoreszieren müßte. Hierzu würde eine maschinelle Detektion benötigt. Ein geeigneterer Weg ist es, den Fluoreszenzmarker mit einem derart ausreichendem Abstand von den Farbstoff-Chromophoren, nämlich einem deutlich größeren Abstand als dem Förster-Radius, der mit minimal ca. 30 Å angesetzt werden kann, zu positionieren, daß als Konsequenz keine Fluoreszenzlöschung durch Energieübertragung erfolgen kann. Ein ausreichender Abstand wäre bereits dann gegeben, wenn der Farbstoff nicht unmittelbar in der Nähe der bindenden Arme des Antikörpers angebracht ist, sondern in weiter entfernten Antikörper-Strukturbereichen. Hierfür stehen geeignete Verknüpfungs-Verfahren zur Verfügung.

Ein zweiter, wichtiger Punkt betrifft die Auswahl einer geeigneten Fluoreszenz-Wellenlänge für das Fluoreszenz-Antikörper-System. Der hierfür zur Verfügung stehende Spektralbereich wird durch die übliche Behandlung von z.B. Textilfasern mit optischen Aufhellern (Textil-Vorbehandlung oder auch aus Waschmittelzusätzen) stark eingeschränkt. Diese fluoreszieren violett bis blau, so daß ein Fluoreszenzmaximum bei ca. 435 nm angesetzt werden kann. Da ein genügender Sicherheitsabstand zu den Emissionen der Aufheller eingehalten werden sollte, ist auch der grüne Spektralbereich zu meiden. Die Fluoreszenzmarker sollten daher vorzugsweise gelb, orangerot oder rot fluoreszieren.

Bevorzugt ist jedoch auch der langweilig rote Spektralbereich zu meiden, weil die Empfindlichkeit des menschlichen Auges bzw. jene von Photomultipliern bei diesen Wellenlängen stark abnimmt. Ganz besonders bevorzugt ist daher eine langweilige Grenze der Fluoreszenz zwischen 650 und 700 nm.

Für eine maschinelle Fluoreszenz-Immunodetektion wäre eine spektrale Zerlegung die geeignete Methode, um das Fluoreszenz-Antikörper-Signal vom Fluoreszenz-Untergrund, bedingt durch die optischen Aufheller, zu separieren. Für eine visuelle Beurteilung der Immunofluoreszenz stört ebenfalls der durch die optischen Aufheller erzeugte Fluoreszenz-Untergrund. Dieser kann hier durch einem Farbfilter mit einer langweiligen spektralen Kante ("Kantenfilter") bei 450 bis 500 nm ausgeblendet werden. Für ein einfaches Routine-Analyseverfahren reicht aber bereits die Betrachtung durch eine bevorzugt gelb gefärbte Kunststoff-Folie völlig aus.

Soll der Nachweis der Immuno-Fluoreszenz mit einer hohen Empfindlichkeit erfolgen, so stört jeglicher fluoreszierender Untergrund der Faser oder des Polymers, der produktionsbedingt oder durch Verunreinigungen hervorgerufen werden kann. Dieses Problem kann im wesentlichen durch den Einsatz von Fluoreszenz-Markern mit vergrößertem Stokes-Shift behoben werden. Eine derartige Vergrößerung des Stokes-Shift ergibt sich aus speziellen strukturellen Erfordernissen. Diese Erfordernisse sind im allgemeinen bei zufällig vorliegenden Substanzen nicht gegeben, weswegen bei diesen gewöhnlich ein "normaler" Stokes-Shift vorgefunden wird. Die Fluoreszenz des Markers kann dann mit üblichen optischen Filtern auf an sich bekannte Weise von der Fluoreszenz des Untergrunds abgetrennt werden.

In einer weiteren bevorzugten Ausführungsform werden unter den Textilfarbstoffen Dispersionsfarbstoffe, die typischerweise auch einen potentiell allergenen Charakter besitzen, verfahrensgemäß nachgewiesen. In einer keinesfalls abschließenden Aufzählung seien beispielsweise Dispersions-Blau 124, Dispersions-Blau 3, Dispersions-Blau 7, Dispersions-Gelb 3, Dispersions-Gelb 9, Dispersions-Orange 3, Dispersions-Orange 76, Dispersions-Rot 1 und Metholmelamin als nachzuweisende Stoffe genannt.

In einer weiteren bevorzugten Ausführungsform werden unter den Textilfarbstoffen Farbstoffe mit einer Azo-Gruppe ("Azofarbstoffe"), die ggf. auch einen potentiell allergenen Charakter besitzen können, verfahrensgemäß nachgewiesen.

Die Analytik von Azo-Textilfarbstoffen hat darüber hinaus durch das Verbot bestimmter Vertreter durch die 2. bis 4. Änderung der Bedarfs-gegenständeverordnung eine besondere Bedeutung erlangt. Da anzunehmen ist, daß das Verbot auf weitere Azofarbstoffe ausdehnt werden wird, ist allgemein ein hoher Bedarf an analytischer Kapazität für die Identifizierung solcher Farbstoffe gegeben. Es werden also zum einen Nachweismethoden benötigt, mit denen bestimmte Azofarbstoffe in gefärbten Textilien identifiziert werden können, und zum anderen werden Methoden benötigt, mit denen die Azo-Gruppierung als Substruktur direkt nachgewiesen werden kann. Der letzere Weg ist deshalb von besonderer Bedeutung, weil weit mehr als 10.000 verschiedene Azofarbstoffe bekannt sind. Die Bereitstellung und Anwendung von spezifischen Bestimmungsverfahren für jeden dieser Azofarbstoffe würde einen außerordentlich großen technischen Aufwand bedeuten. Erschwerend kommt hinzu, daß nicht nur problemlos neue Azofarbstoffe entworfen und synthetisiert werden können, sondern in der Technik häufiger auch Azofarbstoffe als Reaktivfarbstoffe eingesetzt werden, die kovalent mit der Faser verbunden sind und daher nicht extrahiert oder anderweitig desorbiert werden können. Eine Identifizierung solcher Faser-Farbstoff-Konjugate erfordert für übliche Bestimmungsverfahren Spaltungen von chemischen Bindungen, die weitere Unsicherheiten der Analyseverfahren mit sich bringen.

Die Entwicklung eines universellen Analyseverfahren für die Identifizierung von Azofarbstoffen ist daher unbedingt wünschenswert. Bevorzugt sind erfindungsgemäß daher Verfahren, bei denen Antikörper eine hohe Selektivität gegenüber Molekül-Teilstrukturen von Azofarbstoffen aufweisen. Dies wird erfindungsgemäß durch den Einsatz von Antikörpern gegen die inhärente Azo-Gruppe von Azofarbstoffen als antigene Substanz möglich. Derartige verfahrensgemäß eingesetzte Antikörper stellen damit Universal-Reagenzien für Azofarbstoffe dar.

Mit der Bindungsstärke der Antikörper kann die analytische Empfindlichkeit für den Nachweis der Azogruppe eingestellt werden. Dies ist für den praktischen Einsatz eines Testsystems wichtig, da man je nach Erfordernissen eine mehr oder weniger hohe Empfindlichkeit benötigt. Über die Kreuzreaktivitäten läßt sich dann mit einem oder wenigen Antikörpern die Klasse der Azofarbstoffe erfassen. Es ist dabei je nach Erfordernissen günstig, entweder nur einen Antikörper einzusetzen und Unterschiede in den Kreuzreaktivitäten in Kauf zu nehmen oder die Unterschiede durch die Verwendung mehrerer Antikörper zu nivellieren, die den spezifischen, leicht unterschiedlichen Bindungsverhältnissen um die Azo-Gruppe herum Rechnung tragen.

In einer anderen bevorzugten Ausführungsform werden Antikörper eingesetzt, die neben der Azo-Gruppe noch andere Molekül-Teile als Bestandteil der Erkennungsstruktur, dem Epitop, benötigen. Diese weiteren antigenen Molekülstrukturen sind dann für einzelne Azofarbstoffe oder für Subgruppen von Azofarbstoffen charakteristisch. Mit solchen Antikörpern lassen sich also bestimmte Azofarbstoffe identifizieren und quantitativ bestimmen.

In einer besonders bevorzugten Ausführungsform werden Antikörper hierfür mit einem Fluoreszenzmarker über Haupt- oder Nebenvalenzen gekoppelt. Bevorzugt handelt es sich um Marken mit gelbem, orangerotem oder rotem Emissionssignal. Die Fluoreszenzmarker können direkt mit dem Nachweis-Antikörper oder aber an einem Sekundärantikörper, der gegen den Nachweis-Antikörper gerichtet ist, verbunden sein. Um einen großen Abstand zwischen Chromophor und Fluoreszenzmarker herzustellen, befindet sich der Marker in einem vom Paratop des Antikörpers abgewandten Bereich.

Die vorliegenden Erfindung wird durch die nachfolgenden Figuren näher erläutert:

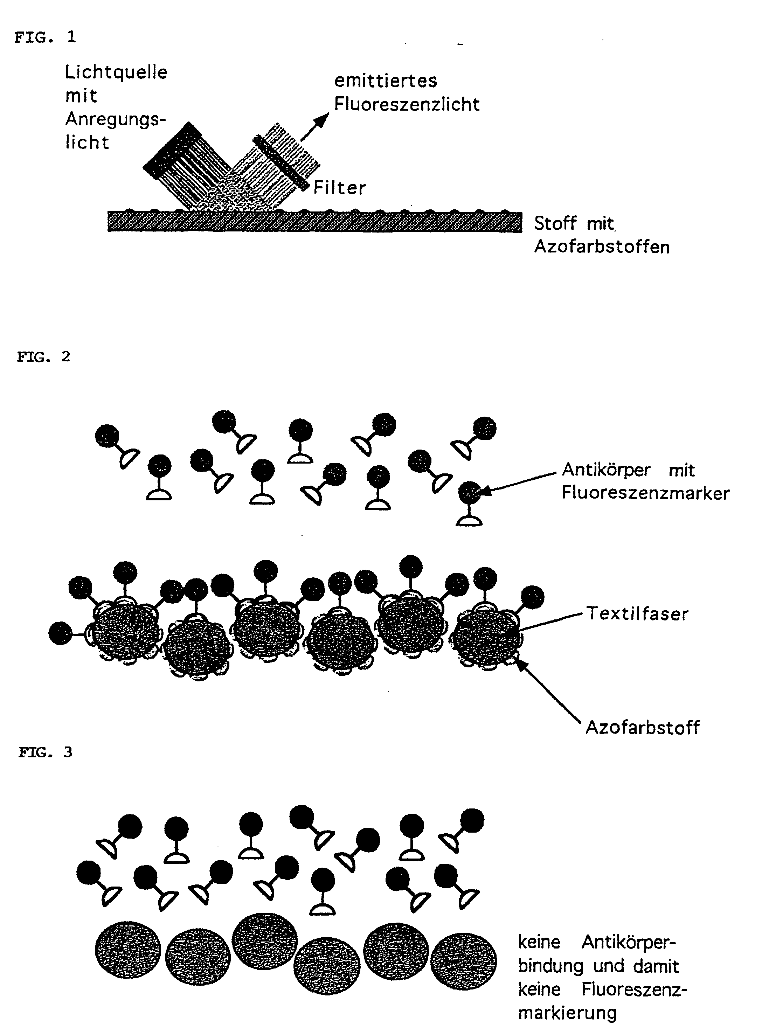

**Figur 1** zeigt schematisch den Nachweis von Antikörpern mit Hilfe von fluoreszenzmarkierten Antikörpern. Hierzu wird Licht einer bestimmten Anregungswellenlänge eingestrahlt. Nach Anregung der Fluoreszenzmarker emittieren diese Fluoreszenzlicht, das gefiltert wird, um unspezifische Signale zu eliminieren. Die Fluoreszenzstrahlung des Markermoleküls kann dann unmittelbar detektiert werden.
**Figur 2** zeigt schematisch die Bindungsreaktion eines spezifischen Antikörpers an ein Antigen auf einer Textilfaser. Im dargestellten Fall handelt es sich um einen fluoreszenzmarkierten Antikörper, der gegen Azofarbstoff gerichtet ist. Nach der spezifischen Bindung des Antikörpers an den Azofarbstoff auf der Faser kann die Detektion erfolgen.
**Figur 3** zeigt schematisch das Verhalten von Antikörpern, die keinen Epitop auf der Faser erkennen. In diesem Fall unterbleibt das Fluoreszenzsignal des Antikörpers auf der Faser.

Die erfindungsgemäßen immunochemischen Nachweisverfahren von Azofarbstoffen auf Textilfasern werden wegen der Verbotsliste für bestimmte Azofarbstoffe dringend benötigt. Darüber hinaus können die erfindungsgemäßen Verfahren aber auch ganz allgemein für den Nachweis von gesundheitlich bedenklichen Substanzen in Fasern oder Polymeren, insbesondere in Textilien, eingesetzt werden. Die erfindungsgemäßen Verfahren können aber auch bei anderen Fragestellungen eingesetzt werden, so etwa bei der Identifizierung bestimmter Inhaltsstoffe von Polymeren oder Fasern zum Nachweis von deren Herkunft. Eine Anwendung des erfindungsgemäßen Verfahrens im kriminologischen Bereich wäre daher gleichfalls denkbar.

## Patentansprüche

1. Verfahren zum Nachweis von Inhaltsstoffen, in welchem die Inhaltsstoffe von Textilfasern und/oder natürlichen oder synthetischen Polymeren mit Hilfe von Antikörpern nachgewiesen bzw. quantitativ bestimmt werden, **dadurch gekennzeichnet, daß** die Inhaltsstoffe direkt auf der Faser von Textilien oder von natürlichen oder synthetischen Polymeren nachgewiesen werden.

2. Verfahren nach Anspruch 1, **dadurch gekennzeichnet, daß** die Oberfläche des zu untersuchenden Substrats zunächst mit Antikörpern abgesättigt wird, die nicht selektiv an den nachzuweisenden Epitop eines Inhaltsstoffs binden.

3. Verfahren nach einem der vorgenannten Ansprüche, **dadurch gekennzeichnet, daß** die unspezifische Antikörper zum Absättigen der Oberfläche von einer anderen Spezies stammen als die spezifischen Antikörper.

4. Verfahren nach einem der vorgenannten Ansprüche, **dadurch gekennzeichnet, daß** die Inhaltsstoffe mit einem oder mehr polyklonalen und/oder monoklonalen Antikörper(n) und/oder einem oder mehr davon abgeleiteten F_{ab}-Fragment(en) nachgewiesen werden.

5. Verfahren nach einem der vorgenannten Ansprüche, **dadurch gekennzeichnet, daß** Antikörper von der Maus, der Ratte, dem Kaninchen, dem Meerschweinchen, dem Schaf, der Ziege, dem Rind, dem Pferd und/oder dem Huhn verwendet werden.

6. Verfahren nach einem der vorgenannten Ansprüche, **dadurch gekennzeichnet, daß** zum Nachweis von Inhaltsstoffen mit Hilfe von Antikörpern radiochemische, enzymatische und/oder Fluoreszenz-Marker eingesetzt werden.

7. Verfahren nach einem der vorgenannten Ansprüche, **dadurch gekennzeichnet, daß** als Antigene unter den Inhaltsstoffen auf Fasern oder Polymeren Textilfarbstoffe, Textilhilfsmittel, deren Beistoffe und/oder Faser- oder Polymerbestandteile mit Hilfe von Antikörpern nachgewiesen werden.

8. Verfahren nach einem der vorgenannten Ansprüche, **dadurch gekennzeichnet, daß** unter den Textilfarbstoffen Dispersionsfarbstoffe mit Hilfe von Antikörpern nachgewiesen werden.

9. Verfahren nach einem der vorgenannten Ansprüche, **dadurch gekennzeichnet, daß** unter den Textilfarbstoffen Azofarbstoffe mit Hilfe von Antikörpern nachgewiesen werden.

10. Verfahren nach Anspruch 9, **dadurch gekennzeichnet, daß** Azofarbstoffe mit solchen Antikörpern nachgewiesen werden, für deren Bindung nur die Azo-Struktur, nicht aber andere charakteristische Molekülteile der Azofarbstoffe relevant sind.

11. Verfahren nach Anspruch 9 oder 10, **dadurch gekennzeichnet, daß** Azofarbstoffe mit Antikörpern nachgewiesen werden, die an die Azo-Struktur und an andere, für den jeweiligen Azofarbstoff charakteristische Molekülteile binden.

12. Verfahren nach einem der vorgenannten Ansprüche, **dadurch gekennzeichnet, daß** Fluoreszenzmarker mit gelber, orangeroter oder roter Fluoreszenz eingesetzt, die auf einem vom Antikörper-Paratop abgewandten Strukturbereich des Antikörpers über Haupt- oder Nebenvalenzen angekoppelt sind.

13. Verfahren nach einem der vorgenannten Ansprüche, **dadurch gekennzeichnet, daß** Azofarbstoffe auf folgenden Materialien nachgewiesen werden: Baumwolle, Wolle oder anderes Tierhaar, so z.B. Roßhaar, Seide, Jute, Sisal, Hanf oder Flachs und deren Umwandlungsprodukte wie z.B. die Viskosefaser, Nitratseide oder Kupferrayon (Reyon) oder synthetische Materialien aus Polyvinylchlorid, Celluloseacetat, Polycarbonate, Polyamide, Polyurethane, Polyimide, Polybenzimidazole, Melaminharze, Silikone, Polyester, Polyether, Polystyrol Polymethylmethacrylat, Polyethylen, Polypropylen, Polyvinylacetat, Polyacrylnitril, Polybutadien, Polychlorbutadien oder Polyisopren bzw. die Copolymeren der genannten Monomeren.

## Claims

1. Method for detecting constituents, in which the constituents of textile fibres and/or natural or synthetic polymers are detected or quantitatively determined with the aid of antibodies, **characterised in that** the constituents are detected directly on the fibre of textiles or of natural or synthetic polymers.

2. Method according to Claim 1, **characterised in that** the surface of the substrate to be analysed is first of all saturated with antibodies which do not bind selectively to the epitope to be detected of a constituent.

3. Method according to one of the preceding claims, **characterised in that** the unspecific antibodies for saturating the surface are from a different species than the specific antibodies.

4. Method according to one of the preceding claims, **characterised in that** the constituents are detected with one or more polyclonal and/or monoclonal anti-body/antibodies and/or one or more F_{ab} fragment(s) derived therefrom.

5. Method according to one of the preceding claims, **characterised in that** mouse, rat, rabbit, guinea pig sheep, goat, cattle, horse and/or hen antibodies are used.

6. Method according to one of the preceding claims, **characterised in that** radiochemical, enzymatic and/or fluorescent markers are used to detect constituents with the aid of antibodies.

7. Method according to one of the preceding claims, **characterised in that**, among the constituents, textile dyes, textile auxiliaries, their ingredients and/or fibre or polymer components are detected as antigens on fibres or polymers with the aid of antibodies.

8. Method according to one of the preceding claims, **characterised in that** dispersion dyes are detected among the textile dyes with the aid of antibodies.

9. Method according to one of the preceding claims, **characterised in that** azo dyes are detected among the textile dyes with the aid of antibodies.

10. Method according to Claim 9, **characterised in that** azo dyes are detected with antibodies, for the binding of which only the azo structure but not other characteristic parts of the molecule of the azo dyes are relevant.

11. Method according to Claim 9 or 10, **characterised in that** azo dyes are detected with antibodies which bind to the azo structure and to other parts of the molecule which are characteristic of the respective azo dye.

12. Method according to one of the preceding claims, **characterised in that** fluorescent markers with yellow, orange or red fluorescence are used, which are coupled to a structural region of the antibody which faces away from the antibody paratope via primary or secondary valencies.

13. Method according to one of the preceding claims, **characterised in that** azo dyes are detected on the following materials: cotton, wool or other animal hair, e.g. horsehair, silk, jute, sisal, hemp or flax and their conversion products, such as viscose fibre, nitrocellulose silk or copper rayon (rayon) or synthetic materials composed of polyvinyl chloride, cellulose acetate, polycarbonates, polyamides, polyurethanes, polyimides, polybenzimidazoles, melamine resins, silicones, polyesters, polyethers, polystyrene polymethyl methacrylate, polyethylene, polypropylene, polyvinyl acetate, polyacrylonitrile, polybutadiene, polychlorobutadiene or polyisoprene or the copolymers of the said monomers.

## Revendications

1. Procédé de détection de substances, dans lequel les substances des fibres textiles et/ou des polymères naturels ou synthétiques sont détectées ou dosées quantitativement, à l'aide d'anticorps, **caractérisé en ce que** les substances sont détectées directement sur la fibre des matières textiles ou des polymères naturels ou synthétiques.

2. Procédé selon la revendication 1, **caractérisé en ce que** la surface du substrat à étudier est d'abord saturée avec des anticorps qui ne se lient pas sélectivement à l'épitope à détecter présent sur une substance.

3. Procédé selon l'une des revendications précédentes, **caractérisé en ce que** les anticorps non spécifiques de saturation de la surface proviennent d'une autre espèce que les anticorps spécifiques.

4. Procédé selon l'une des revendications précédentes, **caractérisé en ce que** les substances sont détectées avec un ou plusieurs anticorps polyclonaux et/ou monoclonaux et/ou un plusieurs fragment(s)-Fab qui en dérivent.

5. Procédé selon l'une des revendications précédentes, **caractérisé en ce que** l'on utilise des anticorps de souris, de rat, de lapin, de cobaye, de mouton, de chèvre, de veau, de cheval et/ou de poulet.

6. Procédé selon l'une des revendications précédentes, **caractérisé en ce que** l'on utilise pour la détection des substances à l'aide d'anticorps des marqueurs radiochimiques, enzymatiques et/ou fluorescents.

7. Procédé selon l'une des revendications précédentes, **caractérisé en ce que** l'on détecte comme antigène parmi les substances sur les fibres ou les colorants textiles polymères, les produits auxiliaires, leurs produits secondaires et/ou des composants de fibres ou de polymères à l'aide d'anticorps.

8. Procédé selon l'une des revendications précédentes, **caractérisé en ce que** l'on détecte des colorants de dispersion parmi les colorants pour textiles à l'aide d'anticorps.

9. Procédé selon l'une des revendications précédentes, **caractérisé en ce que** l'on détecte des colorants azoïques parmi les colorants pour textiles à l'aide d'anticorps.

10. Procédé selon la revendication 9, **caractérisé en ce que** les colorants azoïques sont détectés avec des anticorps tels que, pour leur liaison, seule la structure azoïque est concernée, à l'exclusion des autres fragments caractéristiques de la molécule des colorants azoïques.

11. Procédé selon la revendication 9 ou 10, **caractérisé en ce que** les colorants azoïques sont détectés avec des anticorps, qui se lient à la structure azoïque et à d'autres fragments de la molécule caractéristiques de chaque colorant azoïque.

12. Procédé selon l'une des revendications précédentes, **caractérisé en ce que** les marqueur de fluorescence sont utilisés avec une fluorescence jaune, orange-rouge ou rouge, qui sont couplés à un domaine de structure de l'anticorps autre que l'anticorps-paratope par des valences principales ou secondaires.

13. Procédé selon l'une des revendications précédentes, **caractérisé en ce que** les colorants azoïques sont détectés sur les matériaux suivants; coton, laine ou autres poils d'animaux, par exemple crin de cheval, soie, jute, fibre de sisal, chanvre ou lin et leurs produits de transformation tels que par exemple la fibre de viscose, la soie à la nitrocellulose, la rayonne de cuivre (rayonne) ou des matériaux synthétiques en chlorure de polyvinyle, acétate de cellulose, polycarbonate, polyamide, polyuréthane, polyimide, polybenzimidazole, résine de mélamine, silicone, polyester, polyéther, polystyrène polyméthylméthacrylate, polyéthylène, polypropylène, acétate de polyvinyle, polyacrylnitrile, polybutadiène, polychlorobutadiène ou polyisopréne ou des copolymères des monomères cités.
